# EUROPEAN PATENT APPLICATION

(11) **EP 4 613 321 A1**
(43) Date of publication of application: **10.09.2025**
(21) Application number: 25162008.4
(22) Date of filing: 06.03.2025
(51) Int. Cl.: A61N 1/05, A61N 1/375

(54) **BIOSTIMULATOR HAVING LAMINATE PROBE**

(30) Priority: 07.03.2024 US 202463562660 P; 04.03.2025 US 202519069992
(71) Applicant: Pacesetter, Inc., Sylmar, CA 91342 (US)
(72) Inventor: Kwon, Daniel, Sylmar, CA 91342 (US); McCanless, Jonathan, Sylmar, CA 91342 (US)
(74) Representative: Barker Brettell Sweden AB

(57) **Abstract**

A biostimulator (100) includes a housing (202), a fixation element (106), and a probe (108). The housing (202) includes an electronics compartment (206) containing circuitry. The fixation element (106) is coupled to the housing (202). The probe (108) is coupled to the housing (202) and electrically connected to the circuitry. The probe (108) includes a flexible body (220) extending to a tip (222). The flexible body (220) includes a conductive layer (404) laminated on a substrate layer (406). Other embodiments are also described and claimed.

## Description

### TECHNICAL FIELD

The present disclosure relates to biostimulators. More specifically, the present disclosure relates to leadless biostimulators useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Conventional cardiac pacing systems include leads to penetrate and pace target tissue. The leads carry electrical pacing signals from a remotely situated biostimulator to a pacing site. Leadless cardiac pacemakers incorporate electronic circuitry at the pacing site and eliminate leads, thereby avoiding shortcomings associated with conventional cardiac pacing systems.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. For example, deep septal pacing can target the left bundle branch below the His site. Deep septal pacing has been achieved using a lead in which the electrode penetrates several millimeters into the septum. Pacing thresholds associated with deep septal pacing are potentially lower than with His-bundle pacing, and clinical efficacy of the approach has been demonstrated.

### SUMMARY

Existing pacing leads are not well suited to pacing the deep septal area. Deep septal pacing, e.g., left bundle branch area pacing (LBBAP) can require a pacing electrode to penetrate through a majority of a ventricular septal wall to extend into the left bundle branch or into a left bundle fascicular that resides on a left side of a septum. More particularly, the pacing electrode may be required to penetrate 7-15 mm into the ventricular septal wall. Existing pacing leads lack the ability to control penetration into the target tissue and are either too flimsy or too stiff, susceptible to buckling or promoting tissue trauma. Thus, the leads may not penetrate to an intended tissue or may damage the tissue. Additionally, an effective LBBAP lead may require several attempts at placement before the target region is engaged, and can leave a gaping hole in the septal wall over time. Accordingly, there is a need for a leadless biostimulator or pacing lead having probe configured to penetrate to the deep septal area, possibly after several deployment attempts, and which can flex or comply to the tissue to reduce a likelihood of tissue trauma.

The present invention is defined in the independent claims. Further embodiments of the invention are defined in the dependent claims.

A biostimulator is described. In an embodiment, the biostimulator includes a housing, a fixation element, and a probe. The housing includes an electronics compartment containing circuitry. The fixation element is coupled to the housing. The probe is coupled to the housing and electrically connected to the circuitry. The probe includes a flexible body extending to a tip. The flexible body includes a conductive layer laminated on a substrate layer.

In an embodiment, the probe includes an electrode having the conductive layer to pace or sense target tissue.

In an embodiment, the flexible body has a rectangular cross-sectional profile.

In an embodiment, the tip has a beveled edge.

In an embodiment, the flexible body includes a second conductive layer, and wherein the conductive layer is electrically insulated from the second conductive layer.

In an embodiment, the conductive layer and the second conductive layer are coplanar. In a particular embodiment, the conductive layer is on a first side of the substrate layer, and wherein the second conductive layer is on a second side of the substrate layer.

In another embodiment, the conductive layer and the second conductive layer are non-coplanar.

In an embodiment, the substrate layer is wider than the conductive layer.

A biostimulator lead is described. In an embodiment, the biostimulator lead includes an elongated body, an electrical lead, a fixation element, and a probe. The electrical lead extends through the elongated body. The fixation element is coupled to the elongated body. The probe is coupled to the elongated body and electrically connected to electrical lead. The probe includes a flexible body extending to a tip. The flexible body includes a conductive layer laminated on a substrate layer.

In an embodiment, the probe includes an electrode having the conductive layer to pace or sense target tissue.

In an embodiment, the flexible body has a rectangular cross-sectional profile.

In an embodiment, the tip has a beveled edge.

In an embodiment, the flexible body includes a second conductive layer, and the conductive layer is electrically insulated from the second conductive layer.

In an embodiment, the conductive layer and the second conductive layer are coplanar. In a particular embodiment, the conductive layer is on a first side of the substrate layer, and the second conductive layer is on a second side of the substrate layer.

In another embodiment, the conductive layer and the second conductive layer are non-coplanar.

In an embodiment, the substrate layer is wider than the conductive layer.

A method is described. In an embodiment, the method includes laminating a conductive layer on a substrate layer of a probe. The method includes electrically connecting the conductive layer to circuitry contained within an electronics compartment of a housing of a biostimulator. The method includes mounting a fixation element on the housing proximal to a tip of the probe.

In an embodiment, laminating the conductive layer includes one or more of printing or sputtering the conductive layer on the substrate layer.

In an embodiment, the method further comprising covering the conductive layer with a dielectric layer, and exposing the conductive layer through the dielectric layer.

In an embodiment, the method further comprising cutting the probe out of a laminate sheet having the conductive layer and the substrate layer.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a side view of a biostimulator having a probe, in accordance with an embodiment.
FIG. 3 is a side view of a biostimulator lead having a probe, in accordance with an embodiment.
FIG. 4 is a perspective view of a distal portion of a biostimulator having a helical fixation element, in accordance with an embodiment.
FIG. 5 is a perspective view of a distal portion of a biostimulator having a tined fixation element, in accordance with an embodiment.
FIG. 6 is a perspective view of a probe, in accordance with an embodiment.
FIG. 7 is a top view of a probe having an exposed conductive layer, in accordance with an embodiment.
FIG. 8 is a top view of a probe having several exposed conductive layers, in accordance with an embodiment.
FIG. 9 is a perspective view of a tip of a probe, in accordance with an embodiment.
FIGS. 10 is a cross-sectional view of a probe, in accordance with an embodiment.
FIG. 11 is a cross-sectional view of a probe, in accordance with an embodiment.
FIG. 12 is a cross-sectional view of a probe, in accordance with an embodiment.
FIG. 13 is a cross-sectional view of a probe, in accordance with an embodiment.
FIG. 14 is a flowchart of a method of manufacturing a biostimulator having a probe, in accordance with an embodiment.

### DETAILED DESCRIPTION

Embodiments describe a flexible probe including a conductive layer laminated on a substrate layer. The flexible probe can be incorporated in a biostimulator, such as a leadless pacemaker, or a biostimulator lead. As described below, the flexible probe can be used to perform deep septal pacing of a heart. Deep septal pacing can include left bundle branch area pacing (LBBAP). Deep septal pacing may also include other conduction system pacing (CSP) modalities. The flexible probe may, however, be used in other applications, such as deep brain stimulation. Thus, reference to the flexible probe, biostimulator, or biostimulator lead as being used for deep septal pacing, or deep septal pacing of a particular type, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a probe. Similarly, "proximal" may indicate a second direction opposite to the first direction. Such terms are provided to establish relative frames of reference, however, and are not intended to limit the use or orientation of a biostimulator or biostimulator lead to a specific configuration described in the various embodiments below.

In an aspect, a biostimulator or biostimulator lead includes a flexible probe having a laminated structure. For example, a conductive layer may be printed on a substrate layer. Printing of the conductive layer may be achieved by various manufacturing processes, including screenprinting or sputter coating. The substrate layer can provide structural column strength and/or flexibility to the probe. For example, the substrate layer can be cut out of a sheet of polymer, and can have dimensions such that the probe has a columnar shape that can be both axially stiff and laterally flexible. Accordingly, the printed flex probe can penetrate target tissue axially during implantation, and can bend to comply to pulsating target tissue after implantation.

The printed flex probe can have several advantages over existing electrode implants. As described above, the substrate layer can be sized and shaped to facilitate both penetration of and compliance to the target tissue. For example, mechanical insertion data has been generated to show that embodiments, as described below, can have an average tissue penetration force of 0.07 lbf (0.31 N) to successfully insert into heart tissue. By comparison, an existing leaded body electrode can have a penetration force of 0.30 lbf (1.33 N). Accordingly, the probe can more easily penetrate tissue, and be less traumatic to the implant site. This can allow, for example, the biostimulator and/or the biostimulator lead incorporating the probe to be deployed smoothly and easily several times to find an acceptable location for the probe within the tissue. The substrate layer can also be formed from a polymer, which may be less susceptible to fatigue than existing implantable electrodes.

The printed flex probe can also provide manufacturing advantages. For example, there may be no need for electrode wiring and reflowing of thermoplastics when the conductive layer can be printed to include electrical connections. Printing, e.g., screen printing, may therefore be less labor-intensive than existing electrode implants. Printing processes also include minimal tolerance stack ups, and therefore the printed probe may be accurately sized. At an assembly level, the printed flex probe may be easily inserted into a header assembly and connected to circuitry therein. Such ease may allow for the biostimulator probe to be replaceable, e.g., by removing a first printed probe and inserting a second printed probe. Furthermore, printing is a customizable process that can provide for numerous probe configurations, including contact zones on different sides of the probe, as described below.

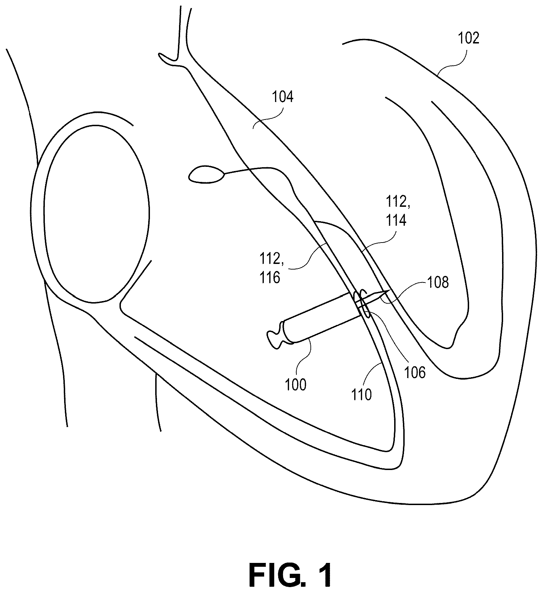

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulator(s) can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a probe 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the probe 108 includes an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a helical fixation element, the transport system can include a docking cap or key at a distal end of the catheter, and the docking cap or key may be configured to engage the leadless pacemaker and apply torque to screw the active engaging mechanism into or out of the tissue.

When the biostimulator 100 is delivered to and anchored into the septum 104 of the heart 102, e.g., by a helical fixation element or a tined fixation element, the probe 108 and/or the fixation element 106 may be positioned for deep septal pacing at respective bundle branches 112 in the septum 104. For example, an active electrode of the probe 108 can be positioned at the left bundle branch 114 in the septum 104. Similarly, the fixation element 106 can be positioned at the right bundle branch 116 in the septum 104. Optionally, one of the elements may be at a bundle branch and the other element may not be at a bundle branch.

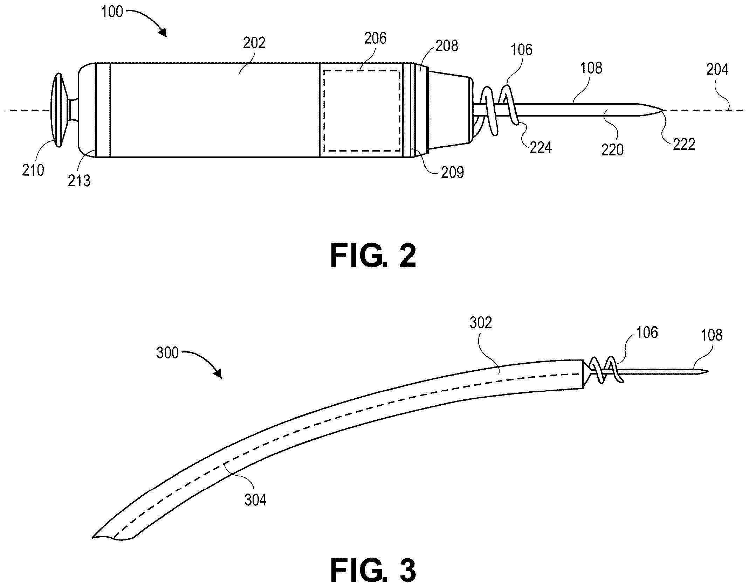

Referring to FIG. 2, a side view of a biostimulator having a probe is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the probe 108 that acts as an active electrode and/or a portion of the fixation element 106 or a housing 202 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 202 having a longitudinal axis 204. The housing 202 can contain a primary battery to provide power for pacing, sensing, and communication, which may include, for example, bidirectional communication. The housing 202 can optionally include an electronics compartment 206 (shown by hidden lines). The electronics compartment 206 can contain or hold circuitry adapted for different functionality. For example, the electronics compartment 206 can contain sensing circuitry (e.g., circuits for sensing cardiac activity from the electrodes), communication circuitry (e.g., circuits for receiving information from at least one other device via the electrodes), pacing circuitry (e.g., circuits for generating pacing pulses for delivery to tissue via the electrodes), or other circuitry. The electronics compartment 206 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

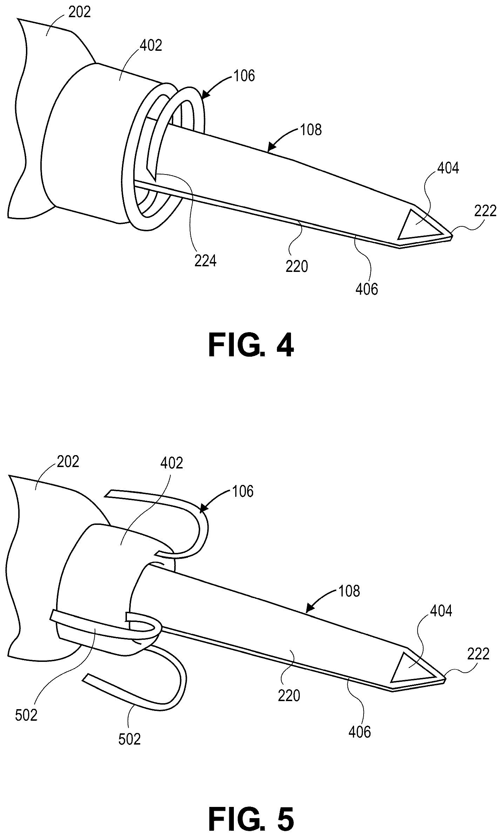

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 202. The fixation element 106 can include a helical fixation element and/or several tines, as described below. More particularly, the biostimulator 100 can include a header assembly having a flange 208 coupled to a distal housing end 209 of the housing 202, and the fixation element 106 can be coupled to, e.g., mounted on, and extend distal to the flange 208. The fixation element 106 can extend about the longitudinal axis 204. For example, in the case of a helical fixation element, the element can spiral about the longitudinal axis 204 to a distal tip 224. In the case of a fixation element 106 having several tines, the tines can be arranged about the longitudinal axis and extend to respective tips (FIG. 5). Accordingly, when the biostimulator 100 is delivered to the target tissue, the tip(s) of the fixation element 106 can pierce the tissue and the housing 202 can be rotated or pushed to affix the fixation element 106 to the target tissue.

In an embodiment, the biostimulator 100 includes an attachment feature 210. The attachment feature 210 can be mounted on a proximal housing end 213 of the housing 202. More particularly, the attachment feature 210 can be mounted on an opposite end of the housing 202 from the fixation element 106 and the probe 108, which as described above, can be coupled to the distal housing end 209 of the housing 202. The attachment feature 210 can facilitate precise delivery or retrieval of the biostimulator 100. For example, the attachment feature 210 can be formed from a rigid material to allow a transport system to engage the attachment feature 210 and transmit torque and/or axial loads to the attachment feature 210 to plunge the fixation element 106 or the probe 108 into the target tissue.

The biostimulator 100 can include the probe 108 coupled to the housing 202. The probe 108 can be coaxially arranged with the fixation element 106 along the longitudinal axis 204. More particularly, the probe 108 can extend axially along the longitudinal axis 204 at a location that is radially inward from the fixation element 106. The probe 108 can have a flexible body extending distally to a tip 222. The tip 222 may be distal to the fixation element 106. For example, a distal fixation tip 224 of the fixation element 106 may be nearer to the housing than the tip 222.

The tip 222 of the probe 108 can be pointed to pierce into the target tissue, as described below. Accordingly, when the fixation element 106 screws into or otherwise engages the target tissue, the probe 108 can also engage the tissue, and the housing 202 can be advanced and/or rotated to cause the tip 222 of the probe 108 to pierce the tissue and anchor the biostimulator 100.

One or more of the fixation element 106 or the probe 108 can include an active electrode, and can electrically communicate with the circuitry contained in the electronics compartment 206. In an embodiment, one or more of the fixation element 106 or the probe 108 are electrically connected to pacing circuitry of the circuitry. Accordingly, the anchored element(s) can electrically communicate with the tissue and can transmit electrical pulses between the tissue and the circuitry of the biostimulator 100.

Referring to FIG. 3, a side view of a biostimulator having a probe is shown in accordance with an embodiment. The biostimulator 100 may include a biostimulator lead 300. The biostimulator lead 300 can be a pacing lead used to engage and pace the septum. For example, the biostimulator lead 300 can include an elongated body 302 having a proximal end (not shown) to physically connect to a biostimulator 100, and a distal portion including the fixation element 106 and the probe 108. The probe 108 can perform pacing or sensing, as in the leadless biostimulator embodiment described above, and the description below therefore applies to the probe 108 of both the biostimulator 100 and the biostimulator lead 300.

In an embodiment, the biostimulator lead 300 includes an electrical lead 304 extending through the elongated body 302. For example, the biostimulator lead 300 can include a cable having a proximal end (not shown) electrically connected to the biostimulator 100. The biostimulator lead 300 can be insulated by the elongated body 302, which jackets the cable, and can transmit electrical pacing impulses from the biostimulator 100 to the probe 108 (or receive electrical sensing signals from the probe 108).

The fixation element 106 and the probe 108 can be coupled to the elongated body 302. Furthermore, one or more of the elements can be electrically connected to the electrical lead 304. For example, the probe 108 can be electrically connected to a wire of the electrical lead 304. Accordingly, pacing impulses delivered through the electrical lead 304 can be received by the probe 108 and transmitted along the flexible body toward the tip, e.g., distal to the fixation element 106.

Referring to FIG. 4, a perspective view of a distal portion of a biostimulator having a helical fixation element is shown in accordance with an embodiment. The biostimulator 100 can include a header assembly that includes the fixation element 106 mounted on a fixation element mount 402. The fixation element mount 402 can be non-conductive. For example, the fixation element mount 402 can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into several turns to form a helical fixation element. The turns can be screwed into the septum 104 to anchor the biostimulator 100 within the heart 102.

In an embodiment, the probe 108, which is coupled to the housing 202, extends in a distal direction to the tip, 222, which can include a distal pacing point. More particularly, the probe 108 can include a flexible probe that extends between the electronics compartment 206 and the distal pacing point 222. The flexible probe can include the flexible body 220 extending in the distal direction through the fixation element mount 402 and the fixation element 106 to the distal pacing point 222 distal to the distal fixation tip 224.

The flexible probe can have a laminate structure. For example, the flexible body 220 can include a conductive layer 404 laminated on a substrate layer 406. The conductive layer 404 can act as an electrode and, as described below, the conductive layer 404 may be exposed to a surrounding environment, e.g., through a dielectric covering. Furthermore, the substrate layer 406 can have a columnar structure that resists axial loading and bends under lateral loading. Accordingly, the substrate layer 406 can support penetration of the tip 222 into target tissue and then comply to bending when inserted within the tissue, e.g., under pulsatile loading from heart beats. The conductive layer 404 can then deliver pacing pulses to the tissue.

Referring to FIG. 5, a perspective view of a distal portion of a biostimulator having a tined fixation element is shown in accordance with an embodiment. The fixation element 106 can be coupled to the housing 202, e.g., mounted on the header assembly, which is mounted on the housing 202. The probe 108 can extend through the header assembly to the tip 222, which may be distal to the fixation element 106. In an embodiment, the fixation element 106 and the probe 108 are configured to plunge into the target tissue. For example, the probe 108 can include the tip 222, and the fixation element 106 can include several tines 502 configured to penetrate axially into the tissue. More particularly, the tines 502 may extend forward in a longitudinal direction when the tines 502 are in a biased state. When released from the biased state, the tines 502 can curl backward into U-shaped structures, as shown in FIG. 5. A longitudinal force can be applied to the proximal end of the housing 202, e.g., the attachment feature 210, to translate the tip 222 and the tines 502 into the target tissue. A stiffness profile of the probe 108 is such that the probe can penetrate the tissue without buckling. Accordingly, the tip 222 and the tines 502 can be pushed forward into the target tissue, and the tines 502 can then curl back to grab the tissue and fix the probe 108 of the biostimulator 100 in place. The tines 502 and the tip 222 of the probe 108 can therefore engage the target tissue without rotating the housing 202 as required for the helical fixation element.

The tissue engagement mechanisms described above facilitate safe redeployment of the biostimulator 100 in a ventricular septum 104. More particularly, the fixation element 106 and the probe 108 can be removed and redeployed several times as needed to achieve stable placement of the pacing tip 222 at the target region. On average, it can take several attempts to position the tip 222 correctly, and the helical or tined elements allow such redeployment to be performed safely. After each deployment, electrical measurements such as pacing capture threshold, impedance, R-wave measurements, paced QRS width, paced QRS morphology changes, or other parameters can be taken to verify suitable placement of the pacing tip 222. When the location is deemed suitable, the biostimulator 100 can be fixed to the septum 104 by the fixation element 106. The pacing tip 222 can therefore be located to deliver pacing impulses to the target region, and the biostimulator 100 can be securely fastened within the heart chamber.

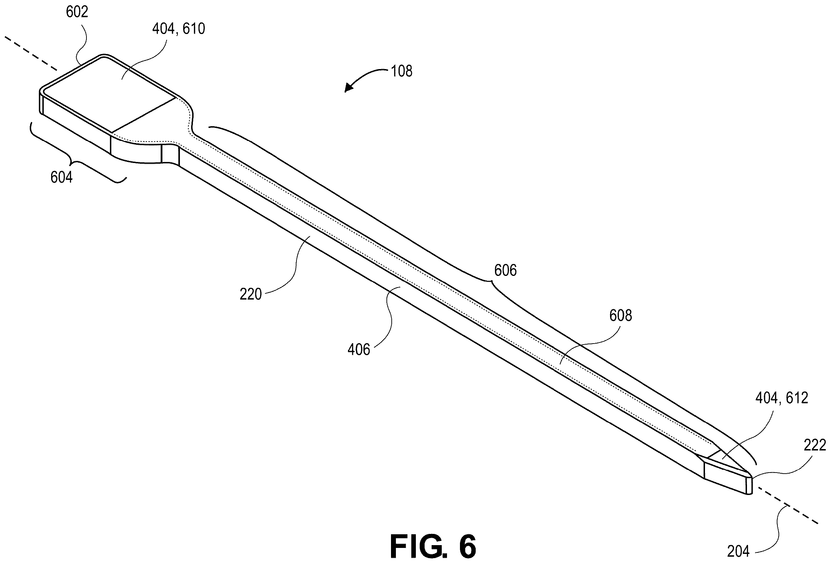

Referring to FIG. 6, a perspective view of a probe is shown in accordance with an embodiment. The probe 108 can include a thin, flat flexible laminate structure. The probe 108 can extend from a proximal element end 602 to the tip 222 in the distal direction. The laminate structure may also be segmented into regions, such as a base region 604, having the proximal element end 602, and a prong region 606, extending distally from the base region 604 to the tip 222. The prong region 606 can include the flexible body 220 and tip 222.

The base region 604 can be sized to facilitate contact and grip of the probe 108. For example, the base region 604 may be inserted into and held by a feedthrough structure of the header assembly. Accordingly, the base region 604 can have a width, transverse to the longitudinal axis 204, which is greater than a width of the prong region 606. The substrate layer 406 forming the base region 604 and the prong region 606 may, however, have a same thickness (perpendicular to the width) over both regions. For example, as described below, the probe 108 can be formed from a polymer sheet, and the regions may have a thickness of the polymer sheet. For example, the substrate layer 406 may have a thickness in a range of 50-1000 microns, such as 250-350 microns, e.g., 300 microns.

The substrate layer 406 can be formed from a polymer having mechanical characteristics that contribute to the axial stiffness and lateral flexibility of the probe 108. In an embodiment, the polymer substrate includes a polyester film. By way of example, the polyester film may be polyethylene terephthalate (PET). The substrate layer 406 can serve as the support structure and base substrate for the electrode material (the conductive layer 404) and, thus, may be any polymer having sufficient strength and compatibility to support and/or receive the conductive layer 404, e.g., in a printing process.

The conductive layer 404 can be formed on top of the heavy gauge insulating film of the substrate layer 406. The conductive layer 404 can span the base region 604 and the prong region 606. More particularly, the conductive layer 404 can have a portion on the base region 604 at which electrical pulses are introduced, and a portion on the substrate layer 406 at the tip 222. The electrical pulses can transmit from the proximal portion to the distal portion through an interconnecting printed trace.

In an embodiment, the conductive layer 404 may be formed from a conductive electrode material, such as conductive ink, graphite, gold, indium tin oxide (ITO), etc. Such materials, and others, are conductive and robust materials, and can be suitable electrode materials for the probe 108. The materials are also suitable for printing processes, such as printing the conductive layer 404 on the substrate layer 406, as described below.

Whereas the substrate layer 406 can provide structural robustness to the probe 108 and the conductive layer 404 serves the function of electrically communicating pacing pulses, the substrate layer 406 may be thicker than the conductive layer 404. The conductive layer 404 thickness can vary based on a manufacturing process used to dispose the conductive layer 404 on the substrate layer 406. For example, when the conductive layer 404 is screen printed graphite on the substrate layer 406, the conductive layer 404 has a thickness in a range of 1-20 microns, e.g., 5 microns. Alternatively, when the conductive layer 404 is sputter coated gold or ITO on the substrate layer 406, the conductive layer 404 has a thickness in a range of 5-500 nm, e.g., 250 nm. In any case, the conductive layer 404 can be thinner than the substrate layer 406.

The laminate structure of the probe 108, including the flexible body 220, can include a dielectric layer 608 on the conductive layer 404. The dielectric layer 608 can cover a portion of the conductive layer 404. For example, the dielectric layer 608 can cover a region of the conductive layer 404 between the base region 604 and the tip 222 of the probe 108. The dielectric layer 608 can insulate the conductive layer 404 from a surrounding environment. More particularly, portions of the conductive layer 404 can be sandwiched and insulated between the dielectric layer 608 and the substrate layer 406 such that the portions do not discharge into the surrounding environment. The insulated conductive layer 404 can therefore transmit electrical pulses distally between the dielectric layer 608 and the substrate layer 406 to pace the target tissue at the tip 222.

The conductive layer 404 may be selectively exposed through the dielectric layer 608. For example, the dielectric layer 608 may cover the outer edges of the conductive layer 404, and can wrap around the edges onto the underlying substrate layer 406. The laterally outward edges of the conductive layer 404 may therefore be insulated from the surrounding environment. Portions of the conductive layer 404 can be exposed to the surrounding environment, however. For example, a contact portion 610 on the base region 604 can be outwardly exposed to allow an electrical feedthrough of the header assembly to make contact and pass a pacing pulse into the conductive layer 404. Similarly, a pacing portion 612 on the tip 222 can be outwardly exposed to pass the pacing pulse outward into the target tissue. A length of the probe 108, including a distance over the base region 604 and the prong region 606 between the exposed contact portions 610, can be insulated from the surrounding environment by the dielectric layer 608 and the substrate layer 406.

The length and a width of the prong region 606 can provide the flexible body 220 having high column strength and lateral flexibility. In an embodiment, the length between the base region 604 and the tip 222 can be in a range of 10-15 mm, e.g., 12 mm. The width, measured laterally from a first lateral side of the substrate layer 406 to a second lateral side of the substrate layer 406, can be in a range of 0.5-1 mm, e.g., 0.75 mm. In combination with the layer thicknesses described above, such dimensions can allow the probe 108 to be sufficiently stiff to plunge into the ventricular septal wall 110 without buckling and, after being plunged into the tissue, to be flexible and compliant when a side load is applied to the tip 222. The probe 108 can therefore function well in both insertion and implanted modes to reduce a likelihood of tissue trauma.

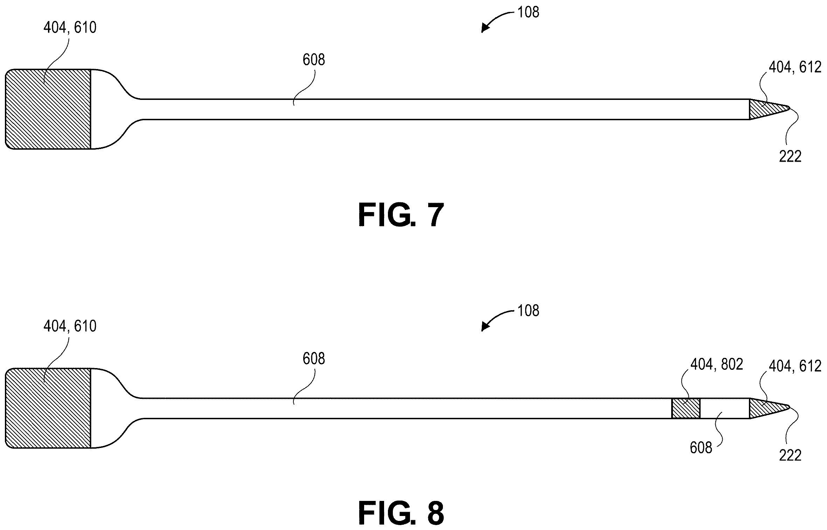

Referring to FIG. 7, a top view of a probe having an exposed conductive layer is shown in accordance with an embodiment. The top view is directed to a first side, e.g., a top side, of the probe 108. The profile includes gray areas, indicating exposed conductive layer portions, and white areas, indicating dielectric layer 608 portions over the conductive layer 404. The proximal exposed area can represent the contact portion 610 that provides a feedthrough connection and the distal exposed area can represent the pacing portion 612 at the tip 222 that can pace/output voltage to cardiac tissue. In an embodiment, a single pacing portion 612 is exposed to the surrounding environment at the tip 222.

Referring to FIG. 8, a top view of a probe having several exposed conductive layers is shown in accordance with an embodiment. The probe 108 can include several pacing portions 612. More particularly, the probe 108 can include the contact portion 610, and may include a second pacing portion 802 along the flexible body 220 between the contact portion 610 and the pacing portion 612. A location of the second pacing portion 802 can be configured to target a predetermined location in the conduction system of the heart 102. For example, the tip 222 and the pacing portion 610 may be plunged to the left bundle branch 114, and the second pacing portion 802 may be spaced apart from the pacing portion 612 to land at the right bundle branch 116. Accordingly, the pacing portions 612, 802 can independently pace respective bundle branches 112.

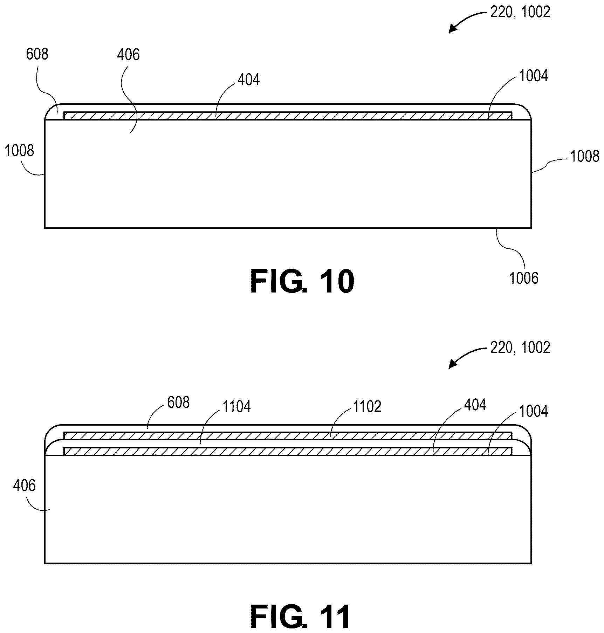

In an embodiment, the pacing portion 612 is part of the conductive layer 404, and the second pacing portion 802 is part of a second conductive layer. For example, the probe 108 can include a second conductive layer, laminated over the conductive layer 404. An embodiment having the several, laminated conductive layers 404 separated by an intermediate dielectric layer is described, for example, below with respect to FIG. 11. The pacing portions can be electrically connected to the contact portion 610, or to respective contact portions. For example, a second contact portion may be layered over the contact portion 610 to allow electrical pulses to be independently transmitted to the second pacing portion 802.

As described below, there are many embodiments of conductive layer configurations. The conductive areas of the probe 108 can be traces, contacts, vias, etc., and can allow for several areas of the probe 108 to be independently and/or simultaneously charged to perform tissue pacing. The variety is enabled by printing of the conductive layer(s) on the substrate layer 406. Printing allows flexibility with respect to where conductive areas are placed on the substrate layer 406, and can be adapted to different pacing geometries and scenarios.

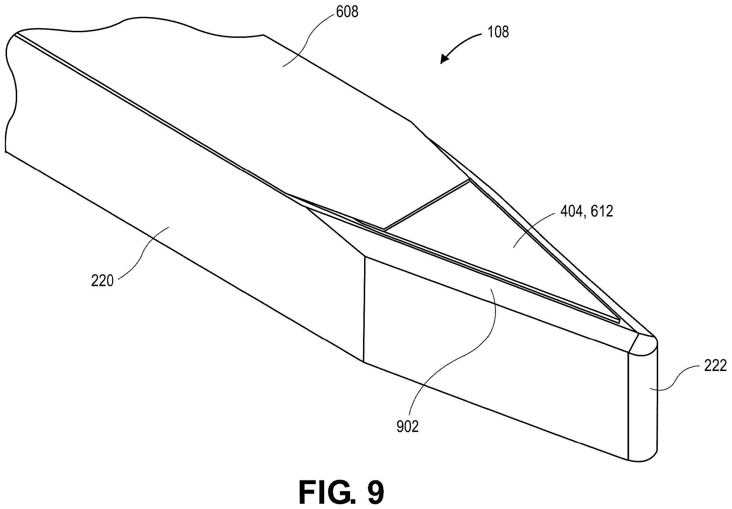

Referring to FIG. 9, a perspective view of a tip of a probe is shown in accordance with an embodiment. The probe 108 can be shaped to enhance tissue penetration. In an embodiment, the flexible body 220 can have converging sidewalls to a form a pointed tip. For example, the distally extending, parallel, sidewalls of the flexible body 220 can be chamfered or tapered to meet at a point. The flexible body 220 of the probe 108 at the tip 222 can have a beveled edge 902. The beveled edge 902 may be formed at the tip 222 of the probe 108. For example, when fabricating the flexible body 220, e.g., by cutting the flexible body 220 having the tip 222 out of a sheet of polymer, the laser beam shape may be controlled to introduce a bevel, curvature, chamber, taper, etc. along an edge of the tip 222. The bevel may be at the tip only. For example, the edge of the flexible body 220 proximal to the tip 222 may have a square, non-beveled edge. The bevel 902 may contribute to tissue penetration. More particularly, by reducing surface area and influencing aspect ratio of the tip 222, the pointed and beveled tip may smoothly puncture organ tissue, e.g., cardiac tissue. An outside surface of most organs has a higher modulus than an inside of the organs and, thus, penetrating the outside surface can require the most puncture force. A sharper tip, produced by the beveled edge 902, may therefore allow penetration to be achieved with lower insertion force, which can reduce a likelihood of electrode buckling during implantation.

Referring to FIG. 10, a cross-sectional view of a probe is shown in accordance with an embodiment. The cross-sectional view can be taken through the flexible body 220, transverse to the longitudinal axis 204, at a location between the base region 604 and the tip 222. In an embodiment, the flexible body 220 has a rectangular cross-sectional profile 1002. The rectangular cross-sectional profile 1002 can reveal a rectangular substrate layer 406, topped by the conductive layer 404 and the dielectric layer 608. The substrate layer 406 can be cut from a sheet, resulting in a rectangular profile having a first side 1004 (e.g., a top side), a second side 1006 (e.g., a bottom side), and several opposing lateral sides 1008. The sides may be orthogonal to each other to form the rectangular profile.

The conductive layer 404 can be disposed on the first side 1004 of the substrate layer 406. The conductive layer 404 may extend over a majority of the width of the substrate layer 406. In an embodiment, however, the substrate layer 406 can be wider than the conductive layer 404. More particularly, the first side 1004 of the substrate layer 406 can extend laterally outward of the conductive layer 404. The wider substrate layer 406 can allow the dielectric layer 608 to encapsulate the conductive layer 404.

In an embodiment, the dielectric layer 608 spans an entirety of the width of the conductive layer 404 and wraps around the laterally outward edges of the conductive layer 404. More particularly, the dielectric layer 608 can completely cover the conductive layer 404 and sandwich the conductive layer 404 against the substrate layer 406. The lateral edges of the dielectric layer 608 can adhere to the substrate layer 406. Accordingly, the dielectric layer 608 can insulate the conductive layer 404 to reduce electrical leakage from the conductive layer 404 to the surrounding environment and ensure transfer of electricity through the conductive layer 404 to the pacing portion 612.

Referring to FIG. 11, a cross-sectional view of a probe is shown in accordance with an embodiment. The probe 108 may have several conductive layers, as described above. In an embodiment, the probe 108 includes a second conductive layer 1102. The second conductive layer 1102 can be insulated from the conductive layer 404. More particularly, the conductive layers 404, 1102 may be independently activated by respective contact portions, and the electrical pulses delivered to each conductive layer can be insulated from each other.

In an embodiment, the conductive layer 404 and the second conductive layer 1102 are non-coplanar. For example, the conductive layer 404 may be disposed on the substrate layer 406, below the second conductive layer 1102. The conductive layer 404 can extend along a first transverse plane, and the second conductive layer 1102 can extend along a second transverse plane above and/or parallel to the first transverse plane. An intermediate dielectric layer 1104 can separate the conductive layers 404. The conductive layer 404 can be encapsulated between the intermediate dielectric layer 1104 and the substrate layer 406. Similarly, the second conductive layer 1102 can be encapsulated between the intermediate dielectric layer 1104 and the dielectric layer 608, which faces outward toward the surrounding environment. The dielectric layers 608, 1104 can be wider than, and extend around the lateral edges, of the conductive layers to fully encapsulate the conductive layers, forming traces along the flexible body 220.

Each of the conductive layers 404, 1102 can be electrically connected to respective pacing portions. For example, the conductive layer 404 shown in FIG. 11 may connect to the pacing portion 612, and the second conductive layer 1102 may be connected to the second pacing portion 802 (FIG. 8). Accordingly, referring again to FIG. 8, the white portion shown proximal to the second pacing portion 802 may be the dielectric layer 608, and the white portion shown distal to the second pacing portion 802 (between the second pacing portion 802 and the tip 222) may be the intermediate dielectric layer 1104. The laminate structure can therefore have several pacing portions facing outward toward the target tissue after implantation.

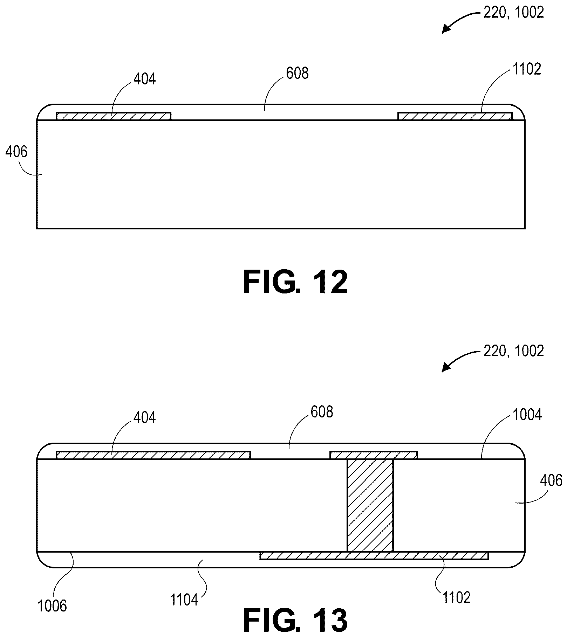

Referring to FIG. 12, a cross-sectional view of a probe is shown in accordance with an embodiment. The conductive layer 404 and the second conductive layer 1102 can be coplanar. For example, the conductive layer 404 and the second conductive layer 1102 may have trace regions disposed side-by-side on the substrate layer 406 within a same lateral plane. The dielectric layer 608 can cover the coplanar conductive layers. The dielectric layer 608 can attach to the substrate layer 406 laterally outward of the conductive layers, similar to FIG. 11, and may adhere to the substrate layer 406 laterally between the conductive layers. Accordingly, the dielectric layer 608 can insulate the conductive layer 404 from the second conductive layer 1102. The insulated conductive layer traces can communicate electrical signals from respective contact portions to respective pacing portions, e.g., the pacing portion 612 and the second pacing portion 802 shown in FIG. 8.

Referring to FIG. 13, a cross-sectional view of a probe is shown in accordance with an embodiment. The probe 108 can include several conductive layers on different sides of the substrate layer 406. For example, the conductive layer 404 may be on the first side 1004 of the substrate layer 406, and the second conductive layer 1102 can be on the second side 1006 of the substrate layer 406. The first side 1004 can be a top side of the substrate layer 406, and the dielectric layer 608 can be layered on the top side to insulate the conductive layer 404. Similarly, the second side 1006 can be a bottom side of the substrate layer 406, and the second dielectric layer 1104 can be layered on the bottom side to insulate the second conductive layer 1102.

In an embodiment, contact portions of the respective conductive layers (top and bottom) can be on a same side of the substrate layer 406. For example, a first contact portion (not shown) can be located on the top side and be electrically connected with the conductive layer 404, and a second contact portion (not shown) can be located on the top side and be electrically connected with the second conductive layer 1102. The electrical connection between the second contact portion and the second conductive layer 1102 may be through a via. More particularly, electrical impulses can travel into the second contact portion, through a trace along the top side laterally offset from the conductive layer 404, and downward through the via to the second conductive layer 1102. The conductive layers can be electrically connected to respective pacing portions at or near the tip 222. Accordingly, the conductive layer 404 can pace a first target, e.g., the left bundle branch 114, and the second conductive layer 1102 can pace a second target, e.g., the right bundle branch 116.

Optionally, the probe 108 can incorporate a therapeutic agent to contact and/or elute into the target tissue after implantation. By way of example, a therapeutic coating, such as a steroid/dexamethasone mixed into a polymer, can be disposed on an outer surface of the dielectric layer 608 and/or the substrate layer 406. The therapeutic coating can contact the target tissue when the probe 108 is inserted therein. The polymer carrier may be a dielectric material, such as polyurethane. The therapeutic coating can therefore insulate the probe 108 from the surrounding environment and can elute therapeutic agent to reduce a likelihood of inflammation of the target tissue into which the probe 108 is implanted.

Use of the biostimulator 100 and, more particularly, the probe 108 to penetrate and pace target tissue can be achieved through several modalities. In an embodiment, a direct puncture modality is used. In the direct puncture modality, the biostimulator 100 is advanced directly toward the target tissue such that the tip 222 of the probe 108 drives directly into the septal wall 110. If the direct puncture modality is not suitable for certain situations, however, then the insertion modality may be altered.

In an embodiment, a needle guided insertion can be used to assist implantation of the probe 108. In such modality, a needle (e.g., a hypotube surrounding the probe 108) can advance through the septal wall 110 to the target tissue. The probe 108 can be delivered through the needle, and the needle may then be retracted back to expose the probe 108 to the target tissue for pacing.

Alternative insertion modalities include incorporating the printed flex probe in a reel-based delivery mechanism. For example, the probe can be rolled up, similar to the rolling of tape, film, or string. Such rolling may be achieved by loading the flexible body 220 on a reel to form a winding. The winding, which includes the loaded probe 108, can be unwound by advancing the tip 222 from the reel toward the target tissue. As the reel rotates to unwind the probe 108, the tip 222 can travel deeper into the septal wall 110. Extension of the probe 108 may be controlled by activation of a toggle to extend a length of the flexible probe. The probe can gradually penetrate the septal wall 110 and reach the left bundle branch area.

In the reel-based modality, the printed flex probe may have a shape memory characteristic. For example, the flexible body 220 may be formed with a shape set, e.g., heat setting process, to preferentially curve or straighten. Accordingly, after unwinding from the reel, the flex probe can straighten from the spiral configuration to insert directly toward the target tissue. In an embodiment, the shape set may be a curved shape, rather than a straight shape. Accordingly, the flexible body 220 of the probe 108 may have a straight or a curved shape when unconstrained.

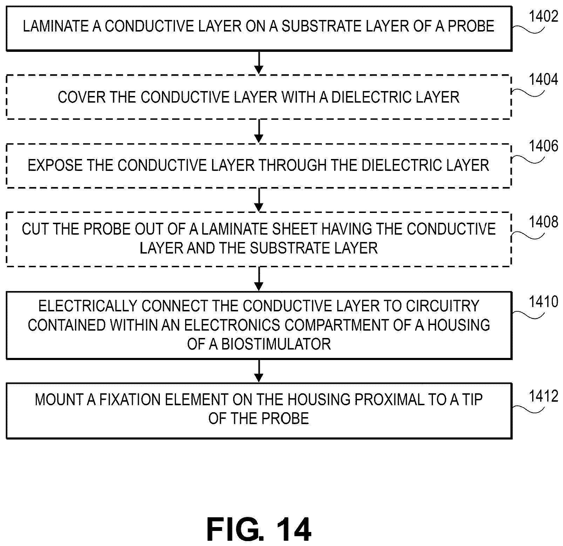

Referring to FIG. 14, a flowchart of a method of manufacturing a biostimulator having a probe is shown in accordance with an embodiment. It will be appreciated that, just as some of the described operations are optional, the sequence of operations is also optional. More particularly, the operations may be performed in a different order than described below. For example, the operation of applying the conductive layer 404 to the substrate layer 406 may be performed before or after cutting the probe 108 profile out of the polymer sheet.

At operation 1402, the conductive layer 404 can be laminated on the substrate layer 406 of the probe 108. Lamination may be achieved using a printing process. For example, the conductive layer 404 can be a graphite layer, and may be screen-printed on the substrate layer 406. Alternatively, the conductive layer 404 may be a gold layer, and may be sputter coated on the substrate layer 406. Other materials may be printed, e.g., deposited, coated, etc.

At operation 1404, optionally, the conductive layer 404 is covered with a dielectric layer 608. The dielectric layer 608 can be deposited or otherwise laminated over the conductive layer 404. The dielectric layer 608 can encapsulate at least a portion of the conductive layer 404 by surrounding the lateral edges of the conductive layer 404 and sandwiching the layer against the substrate layer 406.

At operation 1406, optionally, the conductive layer 404 is exposed through the dielectric layer 608. Exposure of the conductive layer 404 can form the contact portion(s) and/or the pacing portion(s). The exposed portions can face outward, through a window in the dielectric layer 608, to receive or transmit electrical pulses. In an embodiment, the dielectric layer 608 is removed using a masking and etching process, however, other processes, such as laser ablation, may be used.

At operation 1408, optionally, the probe 108 is cut out of a laminate sheet. The laminate sheet can have the conductive layer 404 and the substrate layer 406. For example, the laminate sheet can be a sheet of polymer (providing the substrate layer 406) topped by one or more additional layers (e.g., the conductive layer 404 and/or the dielectric layer 608). The top profile described above can be cut out of the laminate sheet. Cutting may be performed by laser. Laser cutting is a customizable process and, thus, suitable dimensions and geometry can be formed depending on the anatomical requirement, e.g., target tissue depth.

At operation 1410, the conductive layer 404 is electrically connected to circuitry contained within the electronics compartment 206 of the housing 202 of the biostimulator 100. The electrical connection can be made at an assembly manufacturing operation. More particularly, the probe 108 can be inserted into a feedthrough assembly of the biostimulator 100 or the biostimulator lead 300. The feedthrough assembly may, for example, have a slot or recess within which the base region 604 can be located. The probe 108 can be held in place with adhesive, mechanical fasteners, etc.

At operation 1412, the fixation element 106 is mounted on the housing 202. The fixation element 106 can be mounted proximal to the tip 222 of the probe 108 in an assembly manufacturing operation. For example, the fixation element 106 can be loaded onto the fixation element mount 402, can be attached to the elongated body 302, etc. The biostimulator 100 or biostimulator lead 300 may therefore be fabricated for use in tissue pacing.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a housing (202) including an electronics compartment (206) containing circuitry;
a fixation element (106) coupled to the housing (202); and
a probe (108) coupled to the housing (202) and electrically connected to the circuitry, wherein the probe (108) includes a flexible body (220) extending to a tip (222), and wherein the flexible body (220) includes a conductive layer (404) laminated on a substrate layer (406).

2. The biostimulator claim 1, wherein the probe (108) includes an electrode having the conductive layer (404) to pace or sense target tissue.

3. The biostimulator of claim 1 or 2, wherein the flexible body (220) has a rectangular cross-sectional profile (1002).

4. The biostimulator of any one of claims 1 to 3, wherein the tip (222) has a beveled edge (902).

5. The biostimulator of any one of claims 1 to 4, wherein the flexible body (220) includes a second conductive layer (1102), and wherein the conductive layer (404) is electrically insulated from the second conductive layer (1102).

6. The biostimulator of claim 5, wherein the conductive layer (404) and the second conductive layer (1102) are coplanar.

7. The biostimulator of claim 5, wherein the conductive layer (404) and the second conductive layer (1102) are non-coplanar.

8. The biostimulator of claim 6, wherein the conductive layer (404) is on a first side (1004) of the substrate layer (406), and wherein the second conductive layer (1102) is on a second side (1006) of the substrate layer (406).

9. The biostimulator of any one of claims 1 to 8, wherein the substrate layer (406) is wider than the conductive layer (404).

10. The biostimulator of any one of claims 1 to 9, further comprising:
a biostimulator lead (300) having an elongated body (302) coupled to the housing (202); and
an electrical lead (304) extending through the elongated body (302), wherein the fixation element (106) is coupled to the elongated body (302), and wherein the probe (108) is coupled to the elongated body (302) and electrically connected to the electrical lead (304).

11. A method, comprising:
laminating a conductive layer (404) on a substrate layer (406) of a probe (108);
electrically connecting the conductive layer (404) to circuitry contained within an electronics compartment (206) of a housing (202) of a biostimulator (100); and
mounting a fixation element (106) on the housing (202) proximal to a tip (222) of the probe (!08).

12. The method of claim 11, wherein laminating the conductive layer (404) includes one or more of printing or sputtering the conductive layer (404) on the substrate layer (406).

13. The method of claim 11 or 12, further comprising:
covering the conductive layer (404) with a dielectric layer (608); and
exposing the conductive layer (404) through the dielectric layer (608).

14. The method of any one of claims 11 to 13, further comprising cutting the probe (108) out of a laminate sheet having the conductive layer (404) and the substrate layer (406).

15. The method of any one of claims 11 to 14, wherein the probe (108) has a rectangular cross-sectional profile (1002).
